# EUROPEAN PATENT SPECIFICATION

(11) **EP 3 116 448 B1**
(45) Date of publication and mention of the grant of the patent: **11.07.2018**
(21) Application number: 15708537.4
(22) Date of filing: 10.03.2015
(51) Int. Cl.: A61F 2/38, A61F 2/30

(54) **MENISCUS PROSTHESIS**
MENISKUSPROTHESE
PROTHÈSE DE MÉNISQUE

(30) Priority: 11.03.2014 EP 14158887
(43) Date of publication of application: 18.01.2017
(73) Proprietor: Atro Medical B.V., 6534 AT Nijmegen (NL)
(72) Inventor: KOENEN, Jacob, NL-6132 AH Sittard (NL); DAAMEN, Edwin, NL-6121 NP Born (NL); VAN TIENEN, Tony, NL-6511 KC Nijmegen (NL); BUMA, Pieter, NL-6546 VE Nijmegen (NL)
(74) Representative: Nederlandsch Octrooibureau
(86) International application number: PCT/EP2015/054906
(87) International publication number: WO 2015/135907

(56) References cited:
- WO-A1-2012/019248
- WO-A1-2012/168715
- WO-A2-2011/035017
- US-A1- 2011 288 199

## Description

The invention is directed to a meniscus prosthesis and a process for the production of the meniscus prosthesis. The meniscus distributes loads from the femur to the tibia plateau and by its adaptation to the contours of the joint, together with its low friction surface, it provides a smooth nearly frictionless motion of the knee joint. The highly oriented circumferential and radial collagen bundles make the matrix of the meniscus highly anisotropic. Tears (damages) can occur in the meniscus, causing pain and function loss of the knee joint. When tears occur in the meniscus generally a part of the meniscus tissue or the meniscus itself has to be removed. Removal of meniscus tissue may lead to serious osteoarthritic degeneration of the knee joint, especially when a (sub)total meniscectomy was necessary. A meniscus prosthesis would postpone or even prevent other extensive and expensive knee surgeries, such as a total knee replacement. By replacing the ectomized meniscus by an artificial implant the normal joint homeostasis would be restored, the pain could diminish, the function could be restored and further osteoarthritic degeneration could be prevented. Likely this would reduce the cost of healthcare since the number of expensive joint replacement procedures would be reduced.

Meniscus prostheses are known in the prior art.

For example, WO2008/127942 describes a human implantable meniscus device with an anchoring system for locking the device into a bone. Surgically drilled bore channels in the tibial plateau are needed to lock the device. The device is made of a flexible and resilient material. WO2012/168715 describes an implant system for implantation at a joint including an implant device. The implant device comprises an elongate member and a fixation device attached to a body portion. To fix the implant system in the knee joint the fixation device is attached to the tibia by a staple or a screw. For fixation of the elongate member a large channel has to be provided in the tibia bone. The body portion has an anterior end and a posterior end, with the elongate member extending from one end of the body portion for securing the first elongate element to a subject. The implant also comprises a reinforcing structure, such as a piece of ribbon or tape, that runs arounds the outer edge of the body to provide additional reinforcement to the body. The anterior end of the implant comprises a plurality of holes, whilst the posterior section comprises the extension, which has a series of ridges for engaging a screw in a bone hole. Further reinforcement is provided by additional fibres running through the main body of the implant. In WO2011/138045 a non-resorbable meniscus prosthesis is described. The non- resorbable meniscus comprises bone plugs and/or sutures for the fixation of the meniscus prosthesis in the knee joint. A disadvantage of the meniscus prosthesis described in WO2011/138045 is that it takes a relatively long time before the bone plugs are permanently attached due to the relatively slow osseous ingrowth. The body of the meniscus prosthesis is made of one type of biocompatible material. US20130131805 describes an orthopaedic implant comprising different distinct sections, wherein each section comprises a different polymeric material. The orthopaedic implant can be a meniscus implant. The polymeric material preferably is a polyurethane block copolymer.

In WO2008/045807 a meniscus prosthetic device is described comprising a body portion and a fixation member. The body portion and the fixation member form a monolithic structure comprising a flexible polymeric material; preferably a polyurethane. The body portion can comprise a deformation control element comprising a material having increased stiffness relative to the material of the body portion.

It is an object of the present invention to provide a meniscus prosthesis for the human knee joint with an improved shape and improved mechanical properties which is easy to implant in the knee joint.

This object is achieved by a meniscus prosthesis comprising
an arc-shaped meniscus prosthesis body having
a main portion comprising a reinforcing part and
two end portions comprising fixation parts,
wherein the main portion comprises a part made of a first biocompatible, non-resorbable material extending between the two end portions,
wherein the reinforcing part and the fixation parts are made of a second biocompatible, non-resorbable material,
wherein the reinforcing part extends between the fixation parts and
wherein the fixation parts have a through hole, the first biocompatible, non-resorbable material has a tensile modulus of at most 100 MPa as determined by ISO 527-1 and the second biocompatible, non-resorbable material has a tensile modulus of at least 101 MPa as determined by ISO 527-1.

The advantage of the meniscus prosthesis according to the invention is that the meniscus prosthesis is strong enough to withstand the stresses to the prosthesis after implantation and loading of the knee joint and is soft enough to prevent damage to the surrounding cartilage in the knee joint.

A further advantage is that the meniscus prosthesis is easy to implant in the knee joint. Another advantage is that the reinforcing part in the meniscus prosthesis allows fixation of the meniscus prosthesis in the knee joint It is easy to fixate the prosthesis in the knee joint by using sutures or cables in combination with the through holes.

Another advantage is that a strong and durable implant is obtained that can function for years in a human knee joint.

The meniscus prosthesis according to the invention comprises an arc-shaped prosthesis body. The prosthesis body has a main portion and two end portions. The main portion extends between the two end portions and is connected to the end portions.

The main portion of the prosthesis body comprises a part made of a first biocompatible, non-resorbable material having a tensile modulus of at most 100 MPa as determined by ISO 527-1. The tensile modulus is preferably at most 80 MPa, more preferably at most 50 MPa and most preferably at most 25 MPa. The tensile modulus of the first material is for example between 5 and 15 MPa. The tensile test according to ISO 527-1 is described in more detail in the examples.

Preferably, the first biocompatible, non-resorbable material of the main portion is a polymeric material.

The polymeric material of the main portion comprises , for example a hydrogel, for example polyvinylalcohol hydrogels, and/or a thermoplastic material, for example polyacrylonitrile polymers , elastomers, polypropylene, polyethylene, polyetheretherketones (PEEK), silicon rubbers and polyurethanes. Combinations of these thermoplastic materials can also be used.

The materials together with the design of the main portion of the meniscus prosthesis provide the required properties to the meniscus prosthesis body.

Preferably, the polymeric material used in the prosthesis body comprises a polyurethane and more preferably a polycarbonate urethane. Polycarbonate urethanes were the first biomedical polyurethanes promoted for their flexibility, strength, biostability, biocompatibility and wear resistance. These polyurethanes include, but are not limited to the following: Bionate® a polycarbonate-urethane, Bionate® II, a polyurethane with modified end groups, PurSil® a Silicone Polyether Urethane and CarboSil ® a Silicone Polycarbonate Urethane, Elasthane® a Polyether based Polyurethane manufactured by DSM Biomedical Inc. ("DSM"); ChronoFlex® and Hydrothane, manufactured by CARDIOTECH CTE; Tecothante® (aromatic polyether-based polyurethane), Carbothane® (aliphatic polycarbonate-based polyurethane), Tecophilic®. (aliphatic polyether-based polyurethane) and Tecoplast® (aromatic polyether-based polyurethane), manufactured by THERMEDICS; Elast-Eon®, manufactured by AorTech Biomaterials and Texin®, manufactured by Bayer Corporation. The polymeric material used in the prosthesis body can also comprise cross-linked polyurethanes.

The main portion further comprises a reinforcing part made of a second biocompatible, non-resorbable material. The second biocompatible, non-resorbable material has a tensile modulus of at least 101 MPa as determined by ISO 527-1. Preferably, the tensile modulus of the second biocompatible, non-resorbable material is at most 3500 MPa, more preferably at most 3000 MPa, most preferably at most 2000 MPa. For example, the tensile modulus is between 115 and 300 MPa, preferably between 120 and 250 MPa.

Preferably, the second biocompatible, non-resorbable material is a polymeric material. The second biocompatible, non-resorbable material, for example, comprises a thermoplastic material, for example polyacrylonitrile polymers , elastomers, polypropylene, polyethylene, polyetheretherketones (PEEK), silicon rubbers and polyurethanes. Combinations of these thermoplastic materials can also be used.

More preferably the second biocompatible, non-resorbable material comprises a polyurethane and most preferably a polycarbonate urethane. The polyurethanes can be chosen from the same polyurethanes as listed for the first biocompatible, non-resorbable material.

The reinforcing part extends between the fixation parts and is connected to the fixation parts. The reinforcing part can be formed by 1 to 4 parts that are all connected to the fixation parts on both sides. The reinforcing part preferably is one monolithic part. The distance between the fixation parts, following the arc-shape of the meniscus prosthesis body, determines the length of the reinforcing part. The surface area of the reinforcing part is determined perpendicular to the plane in which the arc lies and can be chosen within wide limits by a person skilled in the art based on his technical knowledge. The surface area of the reinforcing part preferably is at least 3.5 mm², more preferably the surface area is at least 7 mm². The reinforcing part can extend along the outer rim of the main portion. The outer rim of the meniscus is the part of the meniscus that forms the outer circumference of the arc-shaped meniscus prosthesis.

Strengthening the meniscus prosthesis has the advantage that deformation of the meniscus in the outward direction is reduced. This has the advantage that the meniscus prosthesis is stable and will be functional for prolonged periods of time when it is implanted in the knee joint.

The first and the second biocompatible, non-resorbable material can comprise additives. Examples of additives are antioxidants, processing aids, lubricants, surfactants, antistatic agents, pigments, dyes and fillers. An additive that is especially preferred is a radiopaque additive, as for example bismuth and bariumsulphate. The addition of a radiopaque additives to the first and/or the second material has the effect that the meniscus prosthesis will be visible at X-ray images of the knee joint. It this way the condition of the meniscus prosthesis after implantation can be monitored. The additives may be present in the typically effective amounts well known in the art, such as 0.001 weight % to 25 weight % based on the total amount of the first or second material.

In some embodiments the meniscus prosthesis body according to the present invention resembles the form of a native meniscus. The meniscus prosthesis body may be a meniscus prosthesis body being of a standard shape, based on a native meniscus, and available in different sizes. Such standard prosthesis may be customized to fit the patient. It may also be possible to make a copy of the patients native meniscus, e.g. with a three-dimensional (3D)-prototyping technique based on tomographic imaging techniques (e.g. CT-scans) or Magnetic Resonance Imaging. An example of a 3D-prototyping technique is rapid prototyping using for example stereo-lithographic sintering (SLS) or fused deposit modelling (FDM). In this way a meniscus body may be directly formed or a mold may be formed according to the negative image of a meniscus body of a patient. Correction of the meniscus prosthesis body or the mold after 3D-prototyping is possible to adapt the meniscus body. For example to adapt the meniscus body better to the patient needs or to amend the meniscus body to remove damage or traces of wear of the native meniscus. The mold may then be used to produce a meniscus body, e.g. with a casting, molding or hot pressing technique. Another example of a 3D-prototyping technique is 3D-printing. An advantage of these embodiments is that it provides more comfort to the patient because once the meniscus prosthesis has been implanted and the trauma has healed, the knee joint comprising the artificial meniscus, closely resembles the knee joint with the original native meniscus. The meniscus prosthesis may behave in a similar way as the original native meniscus. An advantage of using a copy of a meniscus is that these embodiments allow a normal biomechanical motion pattern which may prevent damage of the cartilage in the knee joint. A (nearly) normal behavior of the implant in the knee may provide maximal pain relief.

The prosthesis body of the meniscus prosthesis according to the present invention further comprises two end portions. The end portions of the prosthesis body are the two portions of the prosthesis body where the arc-shaped prosthesis body ends and is narrow.

The end portions of the meniscus prosthesis body according to the present invention comprise fixation parts. As described above the fixation parts are connected to the reinforcing part. This is necessary to obtain a strong fixation of the meniscus prosthesis in the knee joint, wherein the meniscus prosthesis can withstand the forces that are applied to the knee joint during normal use. The fixation parts are made of the second biocompatible, non-resorbable material as described above.

The end portion comprises a fixation part. It should be prevented that the second material of the fixation part is in contact with the cartilage in the knee joint. The second material can be a harder material and can damage the cartilage material over time in case of contact. The fixation parts can be covered with the first material. When the first material is present, preferably at least the sides of the fixation part that will come into contact with the cartilage of the femur and the tibia can be covered with the first material.

The fixation parts have a through hole. The through hole extends from one side of the fixation part to the side opposite thereof. The through hole is meant for fixation of the meniscus prosthesis in the knee joint. When first material is covering the second material of the fixation part the through hole in the fixation part can also extend through the first material.

Sutures can be provided in the through hole. In one embodiment of the invention the through hole has a first portion with a first diameter and a second portion with a second diameter larger than the first diameter. In another embodiment of the invention the through hole comprises an extended part at the side of the meniscus prosthesis that is facing the tibia plateau. The extended part of the through hole is meant to fit into a bore channel made in the tibia plateau. The extended part of the through hole can be made of the first material or of the second material and will fit into the bore channel in the tibia plateau. The extended part of the through hole will prevent damage to the suture(s) after implantation of the meniscus prosthesis by sharp edges of the bore channel in the tibia plateau.

The meniscus prosthesis can be permanently fixed in the knee joint, for example, by sutures. Sutures are preferably made from a non-resorbable material. Combinations of different sutures can be used. The suture can for example be chosen from sutures made of polymeric material like Ultra High Molecular Weight Polyethylene (UHMWPE), for example DSM Dyneema® Purity; polyamide, for example DuPont® Kevlar, Kevlar29, Kevlar49; polyvinylidene fluoride (PVDF);polyester, for example Ethibond Excel ® and nylon. Also sutures from other materials can be used; for example from metal like stainless-steel; titanium and nickel-titanium (Nitinol). Other suitable sutures can for example be made of ceramic material or carbon fibers. Preferably, the through holes in the meniscus prosthesis each comprise at least one suture.

More preferably, the suture is a metal suture. Most preferably, the suture is a stainless-steel suture.

The sutures may be employed in a monofilament or multifilament form as a single strand or a multiple fiber twine. When more than one fiber is used in the suture the fibers can be twisted into a yarn.

Preferably, the suture is provided with a broad section at the end portion of the suture that prevents the suture from slipping through the through holes in the fixation parts. The end portion of the suture can, for example, be a knot. When the through hole in the fixation part comprises two portions with different diameters the end portion of the suture preferably has the same diameter as the portion with the largest diameter in the through hole and is provided in the portion of the through hole with the largest diameter and the main portion of the suture is provided in the portion of the through hole with the smallest diameter.

The invention is also directed to a process for the production of the meniscus prosthesis. The process comprises the following steps:
a. Molding the second material to form the reinforcing part and the fixation parts;
b. Making the through hole in the fixation parts; and
c. Molding the first material to form the part of the main portion of the prosthesis body to enclose the reinforcing part and, optionally, the fixation parts.

Preferably, the reinforcing part and the fixation parts are molded as one piece.

Preferably, the through holes are made through the fixation parts and the first material in the end portions.

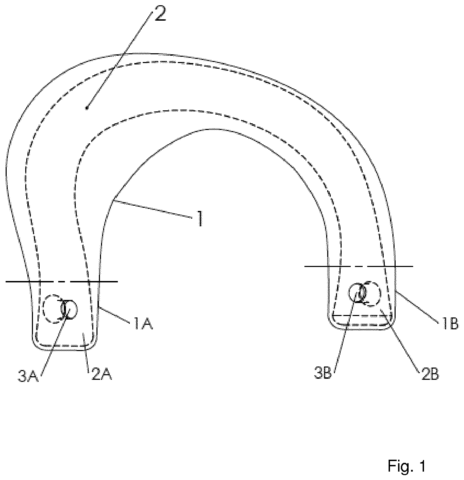

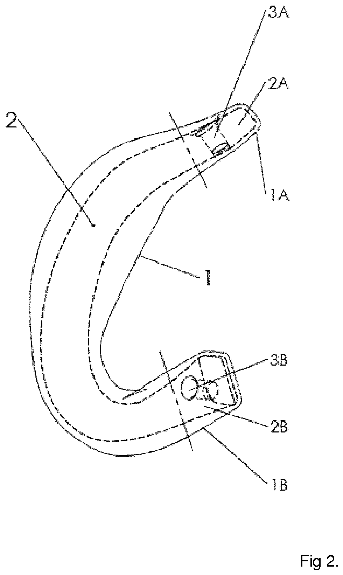

The invention may be used in a method for replacing a native meniscus. The invention is further illustrated by figures 1 and 2. The dotted lines represent parts of the meniscus prosthesis that are located inside the meniscus prosthesis.

Figure 1 is a top view of the meniscus prosthesis and figure 2 is an isometric view of the meniscus prosthesis.

In the figures 1 is the main portion of the arc-shaped meniscus prosthesis body and 1A and 1B are the two end portions.

The reinforcing part is represented by 2 and the fixation parts by 2A and 2B. The fixation parts comprise the through holes 3A and 3B.

Although the invention has been described in detail for purposes of illustration, it is understood that such detail is solely for that purpose and variations can be made therein by those skilled in the art without departing from the spirit and scope of the invention as defined in the claims.

It is further noted that the invention relates to all possible combinations of features described herein, preferred in particular are those combinations of features that are present in the claims.

It is noted that the term 'comprising' does not exclude the presence of other elements. However, it is also to be understood that a description on a product comprising certain components also discloses a product consisting of these components. Similarly, it is also to be understood that a description on a process comprising certain steps also discloses a process consisting of these steps.

The invention will now be elucidated by way of the following examples without however being limited thereto.

### Examples

### Test method

The tensile modulus was determined according to ISO 527-1. The test specimen used was a specimen with the dimensions of the 1 BA type according to ISO 527-2. The test specimen were stamped from injection molded 80x80x2 mm plaques using a special die. The specimens were saturated at least 2 weeks in a physiological buffered salt solution with pH 7.4 at a temperature of 37 ºC prior to tensile testing. The test atmosphere was air with a relative humidity of 100%. The temperature during testing was 37 ºC. The speed of testing was 1 mm/min. The value of the tensile modulus is the average value of 5 test specimens.

### Example 1

The shape of a healthy meniscus was determined by performing MRI scans. A computer model of the shape of a human meniscus was made based on the collected data. An aluminum mold was prepared based on the computer model of an average human meniscus. From the computer model the dimensions for the fixation parts were determined. Together with the necessary through holes the surface area of the cross section of the fixation parts was determined. According to literature, 60 N is known as a normal load that can act upon a human meniscus horn. This load, the surface area of the fixation part and a safety factor of 40% generated stress levels of 5.5 MPa. This stress level was chosen to test the fatigue properties of the second material of the fixation parts.

Bionate® 75D and Bionate® 65D of DSM Biomedical were injection molded in a mold of 80x80x4 mm. From this test piece strips were cut with dimensions 6.6x15.4x4 mm. In the end of these strips a hole was drilled identical in size of the through hole of the meniscus design. The resulting surface area of the test strips was chosen to be equal to the surface area of the actual meniscus fixation parts. One end of the test specimen was held in the grip of a dynamic tensile testing machine. The other end was connected through a pin in hole to the other grip of the tensile machine. Testing was performed according to ISO-527-1. Prior to the start of the test the samples were conditioned in a buffered physiological salt solution with pH 7.4 of 37 ºC until the samples reached a constant weight. This conditioning took about 3 weeks. During the test the whole specimen was kept immersed in the buffered physiological salt solution with pH 7.4 of 37 °C. A sinusoidal tensile load between 0,2 and 11 MPa stress was applied on the 2 mm round pin (1.8 -100 N) for 5 million cycles.

Another test was to determine the loads until break of the horn fixation design according to ISO 527-1.

Result: The test specimen could endure 5 million load cycles and showed permanent deformation of less than 1.5 mm. It was concluded that the material could easily withstand the ambient stress levels in the horn fixation area.

### Example 2

In the meniscus prosthesis good adhesion of the components is important. At the interface of the two materials a "weak spot" in the design could be formed. However it is essential that the two parts adhere strongly to each other to ensure long term performance of the meniscus prosthesis of which this interface is dynamic mechanically loaded.

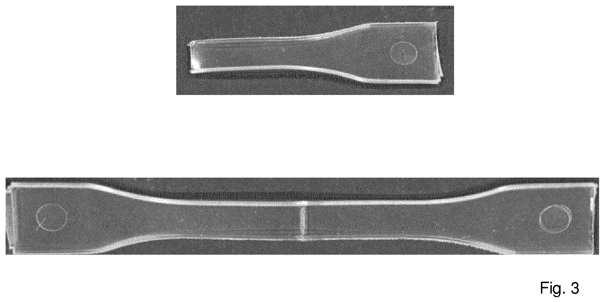

The reference sample was an injection molded 1mm thick test specimen according to ISO 527-2 made from Bionate® II 80A. All other samples were also 1 mm in thickness but contained an adhesion interface that was created by placing half of a test specimen according to ISO 527-2 made from Bionate® II 80A in the mold prior to injection molding of the other using Bionate® II 80A under varying process conditions. These process conditions are given in Table A. In figure 3 the top photo is half of the tensile bar according to ISO 527-1 and the bottom photo is a tensile bar with a visible interface.

Standard molding conditions for the first halves of the tensile bars were:
Melt Temperature 210 °C, Mold temp 50 °C, injection time 0.4 sec, overmolding after 5 min in environment, no preheating, melt residence time 4.4 min, holding pressure 50 MPa.

The standard molding conditions for the reference sample were:
Melt Temperature 210 °C, Mold temp 50 °C, injection time 0.4 sec, no preheating, melt residence time 4.4 min, holding pressure 50 MPa.

Testing was performed according to ISO-527-1. Testing was performed after annealing (24h at 80 °C under nitrogen) and conditioning in a buffered physiological salt solution with pH 7.4 of 37 ºC in a heated chamber kept under 70% relative humidity (RH) conditions until the samples reached a constant weight. 3-5 samples were prepared and tested for each molding condition. All samples broke at the adhesion interface. The test results are given in Table A.

**Table A**

| | Molding parameters | Tensile strength (MPa) average ± sd | Elongatio n at break (%) ± sd |
|---|---|---|---|
| 1 | Standard **without** adhesion interface | 17,4 ± 0,7 | 297 ± 9 |
| 2 | Standard **with** adhesion interface | 18,5 ± 0,8 | 304 ± 10 |
| 3 | 10 °C lower melt temperature | 14,9 ± 0,7 | 264 ± 11 |
| 4 | 20 °C lower melt temperature | 8,2 ± 1,2 | 96 ± 21 |
| 5 | Holding pressure 40 MPa | 22.2 ± 2,0 | 350 ± 17 |
| 6 | Holding pressure 60 MPa | 18,9 ± 4,0 | 309 ± 45 |
| 7 | Long Melt Residence time (4,4→12,2 min) | 19,1 ± 2,0 | 346 ± 21 |
| 8 | Long Injection time (0,4→1,2 sec) | 19,2 ± 1,4 | 310 ± 13 |
| 9 | Long storage (5 min→72 hrs) first half (23°C dry,N2) | 18,3 ± 1,6 | 332 ± 20 |
| 10 | lower mold temperature (50→30 °C) | 14,9 ± 1,6 | 268 ± 23 |
| 11 | preheating first half (23→110 °C for 30 min) | 13,9 ± 4,0 | 267 ± 60 |

| | | | |
|---|---|---|---|
| Sd= standard deviation | | | |

### Observations:

- Maintaining of the normal processing conditions led to a surprisingly strong adhesion at the interface. No loss of strength and elongation properties is observed.
- The values for tensile strength and elongation at break of samples 1 and 2 do not show a large difference. It can thus be concluded that under standard molding conditions the presence of an adhesion interface does not make a lot of difference for tensile strength and elongation at break of a sample.
- When the temperature during molding is lowered with 10 resp. 20 ºC (see samples 2, 3 and 4) the tensile strength and the elongation at break of a sample become worse. It can be concluded that variations in the melt temperature during molding have a strong influence on the properties of the samples.
- When the mold temperature is lowered from 50 to 30 ºC (compare samples 2 and 10) and the mold is preheated at a temperature of 110 ºC (compare samples 2 and 11) this has a clear negative influence on the tensile strength and the elongation at break of the samples.
- Variations in the holding pressure (sample 5 and sample 6), melt residence time (sample 7), storing samples for 72 hrs (sample 9) and longer injection time (sample 8) have a small influence on the on the tensile strength and the elongation at break of the samples when compared with sample 2.

## Claims

1. Meniscus prosthesis comprising
an arc-shaped meniscus prosthesis body having
a main portion (1) comprising a reinforcing part (2) and
two end portions (1A,1B) comprising fixation parts (2A,2B),
wherein the main portion (1) comprises a part made of a first biocompatible, non- resorbable material extending between the two end portions (1A,1B),
wherein the reinforcing part (2) and the fixation parts (2A,2B) are made of a second biocompatible, non-resorbable material and
wherein the reinforcing part (2) extends between the fixation parts (2A,2B),
wherein the fixation parts (2A,2B) have a through hole (3A,3B),
wherein the first biocompatible, non-resorbable material has a tensile modulus of at most 100 MPa as determined by ISO 527-1 and
wherein the second biocompatible, non-resorbable material has a tensile modulus of at least 101 MPa as determined by ISO 527-1.

2. Meniscus prosthesis according to claim 1, wherein the first biocompatible, non- resorbable material comprises a hydrogel and/or a thermoplastic material.

3. Meniscus prosthesis according to claim 1 or 2, wherein the first biocompatible, non-resorbable material comprises a polyurethane.

4. Meniscus prosthesis according to any one of claims 1-3, wherein the second biocompatible non-resorbable material comprises a thermoplastic material.

5. Meniscus prosthesis according to any one of claims 1-4, wherein the tensile modulus of the second material is at most 3500 MPa.

6. Meniscus prosthesis according any one of claims 1-5, wherein the form of the prosthesis body resembles the form of a native meniscus.

7. Meniscus prosthesis according any one of claims 1-6, wherein a three-dimensional (3D)-prototyping technique is used to make a copy of a native meniscus.

8. Meniscus prosthesis according to any one of claims 1-7, wherein the first biocompatible, non-resorbable material and/or the second biocompatible, non-resorbable material comprises a radiopaque additive.

9. Meniscus prosthesis according to any one of claims 1-8, wherein the through hole (3A,3B) has a first portion with a first diameter and a second portion with a second diameter larger than the first diameter,

10. Process for the production of the meniscus prosthesis according to any one of claims 1-9, wherein the process comprises the following steps:
a. molding the second material to form the reinforcing part (2) and the fixation parts (2A,2B);
b. making the through hole (3A,3B) in the fixation parts (2A,2B); and
c. molding the first material to form the part of the main portion (1) of the prosthesis body to enclose the reinforcing part (2) and, optionally the fixation parts (2A,2B).

11. Process according to claim 10, wherein the reinforcing part (2) and the fixation parts (2A,2B) are molded as one piece.

12. Process according to claim 10 or 11, wherein the through holes (3A,3B) are made through the fixation parts (2A, 2B) and the first material in the end portions (1A,1B).

13. The meniscus prosthesis according to claim 1, wherein the second material is enclosed by the first material.

## Patentansprüche

1. Meniskus-Prothese mit
einem bogenförmigen Meniskus-Prothesenkörper, der einen Hauptbereich (1) mit einem Verstärkungsteil (2) und zwei Endbereiche (1A, 1B) mit Fixierungsteilen (2A, 2B) aufweist,
wobei der Hauptbereich (1) ein Teil aufweist, das aus einem ersten biokompatiblen, nicht-resorbierbaren Material hergestellt ist und zwischen den beiden Endbereichen (1A, 1B) verläuft,
wobei das Verstärkungsteil (2) und die Fixierungsteile (2A, 2B) aus einem zweiten biokompatiblen, nicht-resorbierbaren Material hergestellt sind und
wobei das Verstärkungsteil (2) zwischen den Fixierungsteilen (2A, 2B) verläuft,
wobei die Fixierungsteile (2A, 2B) ein Durchgangsloch (3A, 3B) aufweisen,
wobei das erste biokompatible, nicht-resorbierbare Material ein Elastizitätsmodul von höchstens 100 MPa bestimmt nach ISO 527-1 hat und
wobei das zweite biokompatible, nicht-resorbierbare Material ein Elastizitätsmodul von wenigstens 101 MPa bestimmt nach ISO 527-1 hat.

2. Meniskus-Prothese nach Anspruch 1, wobei das erste biokompatible, nicht-resorbierbare Material ein Hydrogel und/oder ein thermoplastisches Material aufweist.

3. Meniskus-Prothese nach Anspruch 1 oder 2, wobei das erste biokompatible, nicht-resorbierbare Material ein Polyurethan aufweist.

4. Meniskus-Prothese nach einem der Ansprüche 1-3, wobei das zweite biokompatible, nicht-resorbierbare Material ein thermoplastisches Material aufweist.

5. Meniskus-Prothese nach einem der Ansprüche 1-4, wobei der Elastizitätsmodul des zweiten Materials höchstens 3500 MPa beträgt.

6. Meniskus-Prothese nach einem der Ansprüche 1-5, wobei die Form des Prothesenkörpers der Form eines natürlichen Meniskus gleicht.

7. Meniskus-Prothese nach einem der Ansprüche 1-6, wobei eine dreidimensionale (3D)-Prototypfertigungstechnik angewendet wird, um eine Kopie eines natürlichen Meniskus herzustellen.

8. Meniskus-Prothese nach einem der Ansprüche 1-7, wobei das erste biokompatible, nicht-resorbierbare Material und/oder das zweite biokompatible, nicht-resorbierbare Material ein röntgenopakes Additiv aufweist.

9. Meniskus-Prothese nach einem der Ansprüche 1-8, wobei das Durchgangsloch (3A, 3B) einen ersten Abschnitt mit einem ersten Durchmesser und einen zweiten Abschnitt mit einem zweiten Durchmesser hat, der größer als der erste Durchmesser ist.

10. Verfahren zur Herstellung der Meniskus-Prothese nach einem der Ansprüche 1-9, wobei das Verfahren die folgenden Schritte umfasst:
a. Gießen des zweiten Materials, um das Verstärkungsteil (2) und die Fixierungsteile (2A, 2B) zu bilden,
b. Herstellen der Durchgangslöcher (3A, 3B) in den Fixierungsteilen (2A, 2B) und
c. Gießen des ersten Materials, um das Teil des Hauptbereichs (1) des Prothesenkörpers so herzustellen, dass das Verstärkungsteil (2) und optional die Fixierungsteile (2A, 2B) umschlossen werden.

11. Verfahren nach Anspruch 10, wobei das Verstärkungsteil (2) und die Fixierungsteile (2A, 2B) in einem Stück gegossen werden.

12. Verfahren nach Anspruch 10 oder 11, wobei die Durchgangslöcher (3A, 3B) durch die Fixierungsteile (2A, 2B) und das erste Material in den Endbereichen (1A, 1B) hergestellt werden.

13. Meniskus-Prothese nach Anspruch 1, wobei das zweite Material von dem ersten Material umschlossen wird.

## Revendications

1. Prothèse de ménisque, comprenant
un corps de prothèse de ménisque en forme d'arc comportant une partie principale (1) comprenant une partie de renfort (2) et
deux parties d'extrémités (1A, 1B) comprenant des parties de fixation (2A, 2B),
dans laquelle la partie principale (1) comprend une partie constituée d'un premier matériau biocompatible, non-résorbable s'étendant entre les deux parties d'extrémités (1A, 1B),
dans laquelle la partie de renfort (2) et les parties de fixation (2A, 2B) sont constituées d'un second matériau biocompatible, non-résorbable, et
dans laquelle la partie de renfort (2) s'étend entre les parties de fixation (2A, 2B),
dans laquelle les parties de fixation (2A, 2B) ont un trou traversant (3A, 3B),
dans laquelle le premier matériau biocompatible, non-résorbable a un module de traction d'au plus 100 MPa tel que déterminé par l'ISO 527-1 et
dans laquelle le second matériau biocompatible, non-résorbable a un module de traction d'au moins 101 MPa tel que déterminé par l'ISO 527-1.

2. Prothèse de ménisque selon la revendication 1, dans laquelle le premier matériau biocompatible, non-résorbable comprend un hydrogel et/ou un matériau thermoplastique.

3. Prothèse de ménisque selon la revendication 1 ou 2, dans laquelle le premier matériau biocompatible, non-résorbable comprend un polyuréthane.

4. Prothèse de ménisque selon l'une quelconque des revendications 1 à 3, dans laquelle le second matériau biocompatible, non-résorbable comprend un matériau thermoplastique.

5. Prothèse de ménisque selon l'une quelconque des revendications 1 à 4, dans laquelle le module de traction du second matériau est d'au plus 3 500 MPa.

6. Prothèse de ménisque selon l'une quelconque des revendications 1 à 5, dans laquelle la forme du corps de prothèse ressemble à la forme d'un ménisque naturel.

7. Prothèse de ménisque selon l'une quelconque des revendications 1 à 6, dans laquelle une technique de prototypage tridimensionnel (3D) est utilisée pour réaliser une reproduction d'un ménisque natif.

8. Prothèse de ménisque selon l'une quelconque des revendications 1 à 7, dans laquelle le premier matériau biocompatible, non-résorbable et/ou le second matériau biocompatible, non-résorbable comprend/comprennent un additif radio-opaque.

9. Prothèse de ménisque selon l'une quelconque des revendications 1 à 8, dans laquelle le trou traversant (3A, 3B) a une première partie avec un premier diamètre et une seconde partie avec un second diamètre plus grand que le premier diamètre.

10. Procédé pour la production de la prothèse de ménisque selon l'une quelconque des revendications 1 à 9, dans lequel le procédé comprend les étapes suivantes consistant à :
a. mouler le second matériau pour former la partie de renfort (2) et les parties de fixation (2A, 2B) ;
b. réaliser le trou traversant (3A, 3B) dans les parties de fixation (2A, 2B) ; et
c. mouler le premier matériau pour former la partie de la partie principale (1) du corps de prothèse destinée à entourer la partie de renfort (2) et, éventuellement les parties de fixation (2A, 2B).

11. Procédé selon la revendication 10, dans lequel la partie de renfort (2) et les parties de fixation (2A, 2B) sont moulées en une seule pièce.

12. Procédé selon la revendication 10 ou 11, dans lequel les trous traversants (3A, 3B) sont réalisés à travers les parties de fixation (2A, 2B) et le premier matériau dans les parties d'extrémités (1A, 1B).

13. Prothèse de ménisque selon la revendication 1, dans laquelle le second matériau est entouré par le premier matériau.
